# EUROPEAN PATENT SPECIFICATION

(11) **EP 4 349 245 B1**
(45) Date of publication and mention of the grant of the patent: **10.12.2025**
(21) Application number: 22200180.2
(22) Date of filing: 07.10.2022
(51) Int. Cl.: A61B 5/00, A61B 5/0533, A61B 5/145

(54) **WRITING INSTRUMENT FOR MEASURING SKIN CONDUCTANCE OF A USER AND METHOD OF OPERATING THE SAME**
SCHREIBGERÄT ZUR MESSUNG DES HAUTLEITWERTES EINES BENUTZERS UND VERFAHREN ZUM BETRIEB DESSELBEN
INSTRUMENT D'ÉCRITURE POUR MESURER LA CONDUCTANCE DE LA PEAU D'UN UTILISATEUR ET SON PROCÉDÉ D'UTILISATION

(43) Date of publication of application: 10.04.2024
(73) Proprietor: BIC Violex Single Member S.A., 145 69 Anoixi (GR)
(72) Inventor: Chrysanthakopoulos, Nikolaos, 145 69 Anoixi (GR)
(74) Representative: Peterreins Schley

(56) References cited:
- EP-A2- 1 190 669
- EP-B1- 3 672 484
- AT-B1- 515 976
- US-B2- 8 934 954

## Description

### Technical Field

The present disclosure relates to a method for measuring skin conductance of a writing instrument's user and a writing instrument for measuring skin conductance of its user.

### Background

Parkinson's disease (PD) is a long-term degenerative disorder of the central nervous system that mainly affects the motor system. The cause of PD is unknown, with both inherited and environmental factors believed to play a role. The symptoms are due to the death of cells in the substantia nigra, a region of the midbrain, leading to a dopamine deficit. The cause of this cell death is poorly understood but involves the build-up of misfolded proteins into Lewy bodies in the neurons.

Its general symptoms include tremor, loss of smell, trouble sleeping, trouble moving or walking, constipation, a soft or low voice, masked face, dizziness or fainting, hunching over. Parkinson's disease begins to become significant for people over 50 years old.

Sudomotor function refers to the autonomic nervous system control of sweat gland activity in response to various environmental and individual factors.

Eccrine sweat is secreted in response to both emotional and thermal stimulation. Eccrine glands are primarily innervated by small-diameter, unmyelinated class C-fibers from postganglionic sympathetic cholinergic neurons. Increases in body and skin temperature are detected by visceral and peripheral thermoreceptors, which send signals via class C and Aδ-fiber afferent somatic neurons through the lateral spinothalamic tract to the preoptic nucleus of the hypothalamus for processing. In addition, there are warm-sensitive neurons located within the preoptic nucleus that detect increases in core body temperature. This triggers the release of intracellular calcium storages and an influx of extracellular calcium which ultimately results in the movement of chloride ion Cl -, sodium ion Na + and water into the duct lumen.

Neuropathy can be caused by several diseases like, autoimmune diseases, diabetes, infections, inherited disorders, tumors, bone marrow disorders, etc. Neuropathy due to phosphorylated alpha-synuclein (Lewy bodies and Lewy neurites), the pathological hallmark of Parkinson disease, has been reported in small peripheral nerves. A study measuring electrochemical skin conductance in hands and feet found that patients with Parkinson disease had sudomotor dysfunction (lower skin conductance) in both hands and feet. This sudomotor dysfunction was associated to myocardial sympathetic denervation and cardiovagal dysfunction even after removing the effect of disease duration and L-Dopa.

These findings support the potential utility of a sudomotor function measurement device as a biomarker of peripheral autonomic nervous system damage in Parkinson disease with Lewy bodies.

SUDOSCAN is a commercially available, non-invasive device, that can detect sudomotor dysfunction. SUDOSCAN evaluates sudomotor function through skin conductance (SC), on the palms of the hands and the soles of the feet where the density of sweat glands is the greatest.

High conductance has been found to be correlated with normal sweat function and healthy nerve innervation (small C-fibers). Low conductance is associated with peripheral or autonomic neuropathy.

The skin conductance response, also known as the electrodermal response (and in older terminology as "galvanic skin response"), is the phenomenon that the skin momentarily becomes a better conductor of electricity when either external or internal stimuli occur that are physiologically arousing.

The underlying technology of skin conductance sensors is based on the established principles of the electrochemical reaction between chloride ions found in sweat and the stainless steel electrodes of the SUDOSCAN system.

A low DC voltage ≤ 4 volts is applied generating a current relative to chloride ion flow supplied by the sweat glands and ducts. An Electrochemical Skin Conductance (ESC) is calculated for each hand and each foot on the basis of the current generated and the voltage applied.

According to FR2887427A1, the chloride ions have the property, in the presence of water, a specific voltage and a pH of less than 7,5, to transform into HCLO and H3O+ by delivering two electrons. On the other hand, for pH values greater than 7,5, the formation of CLO- ions is observed.

These transformations can take place only beyond a critical voltage which depends on the respective concentrations of Cl- and HCLO and the pH value. This voltage is typically about 0,8 V at normal physiological conditions.

Because the skin's stratum corneum acts like an electrical capacitor when a low voltage stimulation is applied, the movement of chloride ions to the electrodes at the surface of the hands and feet can only occur via the sweat ducts, assuring that SUDOSCAN is targeting the underlying sweat gland and its sympathetic innervations.

SC is neither influenced by the Stratum Corneum thickness, nor the sweat conductivity. It is the lateral surface conductance of the wall of the gland which is measured. As chloride is produced by sweat glands, when the latter is stressed by the electric field created by the electrodes, the current generated between the electrodes is representative of the sudomotor function which is impaired by peripheral autonomic neuropathies.

For example, AT515976B1 discloses a device for detecting involuntary muscle contractions in the hand and arm area of a patient, in particular tremor.

Other documents describing the SUDOMOTOR system are FR2994819A1, US8918170B2, US9211080B2, US8934954B2, US10537272B2.

### Summary

The invention is defined by the appended claims.

In a first general aspect, the present disclosure relates to a computer-implemented method for measuring skin conductance of a writing instrument's user, comprising:
applying a DC voltage to user's fingers contacting the writing instrument during a writing session with the writing instrument;
measuring a current generated by reverse iontophoresis following applying the DC voltage;
calculating an average skin conductance over a specific timeframe from the measured current; storing calculated average skin conductance data as historical data;
comparing the average skin conductance to historical data from the same user or to reference data from a group of users; and
providing an indication to the user in case that the skin conductance has decreased and/or the calculation results; and
analyzing health related sweat biomarkers provided by a biosensor of the writing instrument.

In embodiments, the method may comprise measuring at least one of ambient temperature, finger temperature, temperature of an electronic circuit of the writing instrument, and ambient humidity; and calibrating the average skin conductance with at least one of the measured temperatures and the measured ambient humidity.

In embodiments, the DC voltage may be applied in voltage pulses each comprising duration ranges from 0,1 seconds to 10 seconds.

In embodiments, a skin conductance may be calculated based on a number of sequential voltage pulses in a range from 5 to 50.

In embodiments, the indication and/or the calculation results may be provided via a user interface of the writing instrument.

In embodiments, the indication and/or the calculation results may be provided via a wireless communication system to an external device having an interface.

In a second general aspect, the present disclosure relates to a writing instrument for measuring skin conductance of its user, comprising:
at least two electrodes configured to apply a DC voltage to user's fingers contacting the writing instrument during a writing session with the writing instrument;
one or more sensors configured to measuring a current generated by reverse iontophoresis following applying the DC voltage;
a computer system configured to execute the computer-implemented method for measuring skin conductance according to one of the preceding claims;
a storage system configured to store calculated average skin conductance data as historical pressure data; and
a communication system configured to provide an indication to the user in case that the skin conductance has decreased and/or the calculation results;
a biosensor configured to analyze health related sweat biomarkers.

In embodiments, the writing instrument may comprise at least one temperature sensor configured to measure at least one of ambient temperature, finger temperature, temperature of an electronic circuit of the writing instrument; and a humidity sensor configured to measure ambient humidity.

In embodiments, the electrodes are located at a grip area of the writing instrument.

In embodiments, two of the electrodes are the anode and the cathode electrodes during a measurement and wherein a third or more electrodes are passive electrodes and are connected with high impedance configured to measure the potential reached by the user's body.

In embodiments, a gap area made of a non-conductive material is provided between two electrodes.

In embodiments, a surface of the gap area is elevated or receded relative to a surface of the electrodes.

In embodiments, the writing instrument may comprise a user interface configured to receive input from a user and/or to provide information to the user such as the indication and/or the calculation results; and/or a wireless communication system configured to provide the indication and/or the calculation results via an external device having an interface.

According to examples of the present disclosure, early identification of symptoms that may be related to neurodegenerative diseases, like Parkinson's disease, is achieved by measuring finger's sudomotor functions with electrodes and sensors embedded on a smart pen. Sudomotor functions are the autonomic nervous system control of sweat gland activity in response to various environmental and individual factors.

Sensors located at pen's finger position are capable of measuring nerve associated skin conductance regularly when a person uses the pen for handwriting and identifying any consistent changes from normal nerve function, leading to low conductance.

Steps of an example method of the present disclosure, not claimed as such but useful to understand the invention, may be as follows:
User writes regularly with his smart pen.

Most of the times, holds the pen with his three fingers (thumb, index, middle), both when he writes and during airtime (not actively handwriting).

Fingers excrete sweat continuously.

Sweat contains chloride ions.

Smart pen has conductive electrodes in the finger rest position of the body.

Because the skin's stratum corneum acts like an electrical capacitor, when a low DC voltage stimulation (≤4V) is applied, the movement of chloride to the electrodes can only occur via the sweat ducts.

A current is generated, relative to chloride ion flow supplied by the sweat glands and ducts. Active electrodes located on the finger rest position measure skin conductance on the basis of the current generated by the sweat glands and the voltage applied.

During normal nerve function, a baseline skin conductance of the user is established.

In case of nerve dysfunction, a consistently lower skin conductance is measured.

In case of repeated low conductance measurements, system notifies user and recommends further testing by a doctor.

An example system of the present disclosure, not claimed as such but useful to understand the invention, may provide the following features

A smart pen with appropriate electronics and a power source capable of powering the electrodes with DC voltage.

An area with conductive electrodes at the fingers gripping position of the smart pen body, permitting the measurement of skin conductance.

An area with insulating material between the electrodes, to avoid any short circuit between the electrodes.

In examples, a biosensor located on the pen body analyzing lactate or other metabolites found in sweat.

An environmental temperature and humidity sensor at the top of the smart pen for the calibration of the measurements.

Network capabilities for transmitting measurement data.

A smartphone or tablet receiving measurement data.

A software application for the operation of the smart pen and the visualization of the data.

Particular examples of the first to second general aspects can be implemented so as to realize one or more of the following advantages.

In examples, a system is provided to measure skin conductance on the fingers of a user of a smart pen during handwriting without any user action other than holding the pen.

In examples, the present disclosure enables to measure and record skin conductance (SC) over time and identify consistent changes of SC decrease.

In examples, the present disclosure enables to correlate SC changes to a disfunction or degeneration of sweat gland nerves due to peripheral neuropathy and alert the user to seek medical advice.

The present disclosure allows identifying a chronic or degenerative disease in early stages which is very difficult in most cases.

The present disclosure allows to detect the quality of handwriting which may be severely affected when medium to severe symptoms of a chronic or neurodegenerative disease are expressed.

The present disclosure overcomes issues in that testing for and measuring biomarkers related to neurodegenerative diseases is a process performed in a medical point of care (hospital, doctors office) and may require sophisticated equipment.

Sweating or perspiration is the production of fluids secreted by the sweat glands in the skin of mammals. Sweat production is a vital thermoregulatory mechanism used by the body to prevent heat-related illness as the evaporation of sweat is the body's most effective method of heat reduction and the only cooling method available when the air temperature rises above skin temperature.

Two types of sweat glands can be found in humans: eccrine glands and apocrine glands. The eccrine sweat glands are distributed over much of the body and are responsible for secreting the watery, brackish sweat most often triggered by excessive body temperature. The apocrine sweat glands are restricted to the armpits and a few other areas of the body and produce an odorless, oily, opaque secretion which then gains its characteristic odor from bacterial decomposition.

In humans, sweating is primarily a means of thermoregulation, which is achieved by the water-rich secretion of the eccrine glands. Maximum sweat rates of an adult can be up to 2-4 liters per hour or 10-14 liters per day (10-15 g/min·m2).

Sweat is mostly water. Dissolved in the water are trace amounts of minerals, lactic acid, and urea. Although the mineral content varies, some measured concentrations are sodium (0,9 gram/liter), potassium (0,2 g/L), calcium (0,015 g/L), and magnesium (0,0013 g/L).

The fingertips are home to the highest concentration of sweat glands on the body - even higher than in the armpits - and they make sweat constantly, regardless of whether one exercises. We typically don't notice finger sweat because it evaporates almost instantly.

Regarding hand sweat as a disease biomarker, metabolic biomonitoring in humans is typically based on the sampling of blood, plasma or urine. These sampling procedures are often associated with a variety of compliance issues, which are impeding time-course studies. On the other hand, the metabolic profiling of the minute amounts of sweat sampled from fingertips addresses this challenge.

Sweat sampling from fingertips is non-invasive, robust and can be accomplished repeatedly by untrained personnel. The sweat matrix represents a rich source for metabolic phenotyping. The feasibility of short interval sampling of sweat from the fingertips is achieved e.g. in time-course studies involving the consumption of coffee or the ingestion of a caffeine capsule after a fasting interval, in which all known caffeine metabolites as well as endogenous metabolic responses were monitored.

Fluctuations in the rate of sweat production are accounted for by mathematical modelling to reveal individual rates of caffeine uptake, metabolism and clearance.

Sweat has been associated with health and disease ever since it was linked to high body temperature and exercise. It contains a broad range of electrolytes, proteins, and lipids, and therefore hosts a broad panel of potential noninvasive biomarkers.

Proteomic and metabolomic technologies now enable sweat analysis with unprecedented sensitivity and numbers of detected metabolites at the same time. In a study more than 800 unique proteins and 32.000 endogenous peptides were detected in sweat and opened up an exciting field of potential novel, noninvasive biomarkers.

A biosensor that collects and analyses sweat could provide valuable information for user's health.

The present disclosure may employ a biosensor which is an analytical device used for the detection of a chemical substance, that combines a biological component with a physicochemical detector.

A low- cost electronic biosensor can be made e.g. of semiconducting polymer, capable of detecting quickly and with high sensitivity, a wide range of health conditions from body fluids. The sensor can measure the amount of critical metabolites, such as e.g lactate, glucose or cholesterol, that are present in sweat, and when incorporated into a diagnostic device, could allow health conditions to be monitored quickly, cheaply and accurately. The new device has a far simpler design than existing sensors and opens up a wide range of new possibilities for health monitoring down to the cellular level.

A low-cost biosensor may measure levels of lactate, which is useful in fitness applications. But it can be easily modified to detect e.g., glucose or cholesterol by incorporating the appropriate enzymes.

In the example of a semiconductor sensor, researchers used a newly-synthesised polymer that acts as a molecular wire, directly accepting the electrons produced during electrochemical reactions. When the material comes into contact with a liquid such as sweat, tears or blood, it absorbs ions and swells, becoming merged with the liquid. This leads to significantly higher sensitivity compared to traditional sensors made of metal electrodes.

Further, when the sensors are incorporated into more complex circuits, such as transistors, the signal can be amplified and respond to tiny fluctuations in metabolite concentration, despite the tiny size of the devices.

Since the sensor does not consist of metals such as gold or platinum, it can be manufactured at a lower cost and can be easily incorporated in flexible and stretchable substrates, enabling their implementation in wearable or implantable sensing applications.

Regarding writing action, handwriting is the writing action done with a writing instrument, such as a pen or pencil, using the hand. Handwriting includes both printing and cursive styles. Handwriting involves the use of a pen or pencil which the user keeps holding with his hand/fingers even when he does not write. At a writing session the user writes or sketches with a writing instrument. A writing session includes actual handwriting instances and on-air instances in which a tip of the writing instrument is not touching a writing surface.

Significant changes or the deterioration of a person's handwriting may be a symptom or a result of several different diseases.

The normal pen grip may be performed in the following way:
- the writing instrument is held in a stable position between the thumb, index and middle fingers,
- the ring and little fingers are bent and rest comfortably on the table,
- the index finger and thumb form an open space,
- the wrist is bent back slightly, and the forearm is resting on the table,
- the writing instrument is held about 1- 2 cm from the tip.

Certain terms are used in the following manner in the present disclosure:
The expression "digital device" may refer to an electronic device that uses discrete, numerable data and processes for all its operations, and is capable of displaying content to the user. Examples of such a device include but are not limited to: Mobile phones, Laptops, Tablets, Personal computers, Netbooks, iPads, etc.

The expression "connected to" is not limited to a direct connection between two objects (i.e., it is not required that the two objects abut). For instance, a first object and a second object can be indirectly connected through a third object. The invention is defined by the appended claims.

### Description of the Drawings

**Fig. 1** illustrates an overview of components of a writing instrument for measuring skin conductance of its user according to the present disclosure.
**Fig. 2** illustrates a process flow diagram of a writing instrument according to the present disclosure.
**Fig. 3** illustrates a perspective view of a writing instrument according to the present disclosure.
**Fig. 4** illustrates a sectional view of an electrode arrangement of a writing instrument according to the present disclosure.
**Fig. 5** illustrates a method flow chart for measuring skin conductance of a writing instrument's user according to the present disclosure.

### Detailed Description

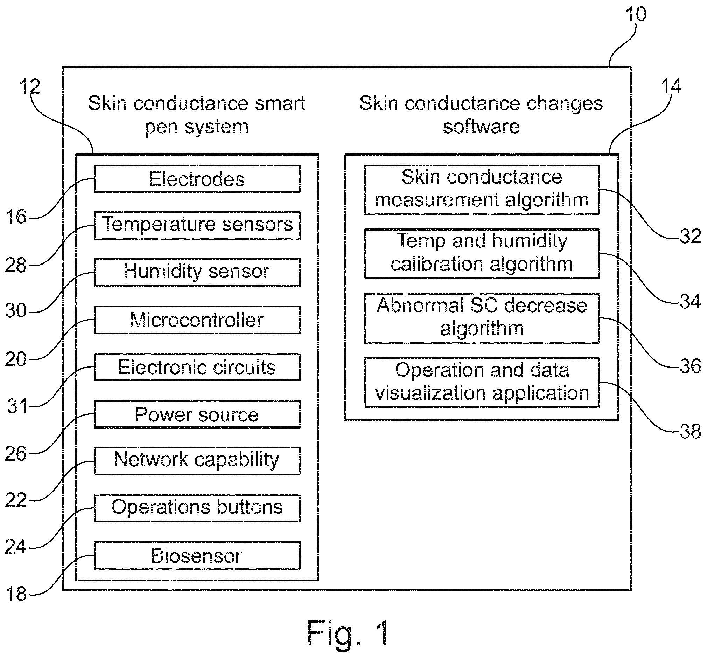

**Fig. 1** shows an overview of components of a writing instrument 10 for measuring skin conductance of a writing instrument's user according to the present disclosure. The writing instrument 10 can be named smart pen, digital writing instrument or digital device.

The writing instrument 10 includes a sweat gland innervation assessment system which is capable of measuring the skin conductivity due to sudomotor effects of the fingers of the user and evaluating if there is any abnormality in the sweat gland innervation.

The sweat gland innervation assessment system includes two subsystems i.e., a skin conductance (measurement) smart pen system 12 and a skin conductance changes software 14.

The skin conductance measurement smart pen system 12 is capable of measuring the skin conductance of the fingers of the user, when the user grips the writing instrument 10 to perform handwriting and/or sketching action or just holds the smart pen.

The skin conductance measurement smart pen system 12 includes the following elements:
One or more electrodes 16 capable of applying potential to user's fingers while holding a writing instrument 10.

The electrodes may be located at the body of the writing tool, more specifically at the lower part of the pen's body where usually a user holds the pen.

In embodiments, electrodes may extend from the lower part to the middle part of the writing tool to cover other pen gripping styles.

In embodiments, electrodes may be located from 0,5 cm to 7 cm from the tip of the writing instrument, more specifically from 2 cm to 5 cm.

In embodiments, the number of the electrodes may range from 2 to 10, more specifically limited to 3, one for each of the finger usually used by a user to hold a pen.

In embodiments, two of the electrodes during a measurement are considered the active electrodes. One of the electrodes may be considered as the anode, a second electrode may be considered as the cathode.

In embodiments, a third or more electrodes are considered passive electrodes and may be connected with high impedance, directly or indirectly via an operational amplifier and are suitable for measuring the potential reached by the body.

In embodiments, the material of the electrodes may include at least one of stainless steel, Gold, Silver, Copper, Graphite, Titanium, Brass, and Platinum.

In embodiments, the width of each electrode may range from 3 mm to 20 mm

In embodiments, the length of each electrode may range from 0,5 cm to 5 cm.

In embodiments, the area of each electrode may range from 0,15 cm² to 10 cm².

In embodiments, the electrodes may be flat or may be curved to accommodate the shape of the writing tool.

In embodiments, the electrodes may be placed on facets of a multifaceted writing instrument. For example, there may be 3 electrodes, each one placed on a facet of a 3 faceted writing instrument, each of the facets used as the grip areas of the 3 fingers used to hold the pen to facilitate finger positioning.

In embodiments, the DC voltage provided by the electrodes ranges from 0,5 V to 10 V.

In embodiments, the duration of each voltage pulse ranges from 0,1 sec to 10 sec.

In embodiments, the voltage may be applied to any of the electrodes of the writing instrument, in the same order or alternatively and sequentially by a switching circuit.

Using the electronic switching circuit, sequential pulses of the DC voltage can be applied on a pair of electrodes, with the value being able to be positive or negative by simple polarity inversion.

In embodiments, the voltage may be applied in successive waves, where in each wave the voltage may vary by a specific value.

In embodiments, the value of variation of the voltage of the successive waves may range from 0,05 V to 1 V.

In embodiments, the number of sequential voltage pulses used to calculate a skin conductance measurement may range from 5 to 50.

In embodiments, the applied voltage on the anode induces, through reverse iontophoresis, a voltage on the cathode and generates a current.

In embodiments, the intensity of the current may range from 0,01 mA to 10 mA.

In embodiments, the current carried across the gland by chloride ions and protons, is going through the body between anode and cathode and is directly related to skin conductivity.

In embodiments, the system is thus able to measure the evolution of the current which passes through the fingers between the two active two electrodes when the DC voltage is applied.

In embodiments, the current signal is acquired through an acquisition circuit with a sampling frequency of between 50 Hz and 1000 Hz.

Between two subsequent electrodes 16 located on the pen, there may be a gap made of a non-conductive material that acts as an insulator. This insulating material protects the involuntary contact of the same finger with two adjacent electrodes and the potential short-circuit of the system.

The material of the gap may be at least one of ABS, PP, PS, rubber, and wood.

In embodiments, the width of the gap insulating material may range from 1 mm to 10 mm.

In embodiments, the length of the insulating gap may range from 0,5 cm to 5 cm.

In embodiments, the gap surface may be at the same level with the electrodes surface, or more specifically may be more elevated than the electrodes surface or may form a recess relative to the electrodes surface.

In embodiments, the height of the elevation/recess of the insulating gap may range from 1 mm to 10 mm.

In embodiments, the surface of the insulating gap may be flat or may be curved or may be pointy in order to make the user limit his finger position in one of the electrode areas of the writing instrument.

In embodiments, instead of electrodes 16, one or more biosensors 18 with similar dimensional characteristics could be located on the smart pen body, at the finger gripping positions.

A biosensor 18 could permit the absorption of fingers sweat, during writing action through an absorbing material and a microfluidic network and the analysis of lactate or other metabolites found in the sweat. The biosensor 18 could provide useful health related metrics to the user.

A microcontroller 20 (MCU) may process and control all the sensors, circuits and functions of the writing instrument 10 and may be of conventional wearable type or may be able to perform advanced AI processing. It may contain a flash memory module.

The microcontroller 20 may be a conventional ultra- low power MCU suitable for wearable applications such as but not limited, a 16 or 32-bit-ARM MCU. Alternatively, the microcontroller 20 may be based on a platform such as customizable single-chip ASIC AI or be based on a RISC-V AI architecture. The microcontroller 20 may have a custom operating firmware.

The skin conductance measurement smart pen system 12 may further include network capability 22. Wireless connectivity is provided for the smart pen to interact with other devices. The network capability 22 and the other device(s) support at least one of
a. Wi-Fi,
b. ANT+,
c. Bluetooth Low Energy (BLE),
d. IEEE 802.15.4.

The skin conductance measurement smart pen system 12 may further include operation buttons 24 to control voltage and smart pen functions. The control buttons can be located on the one side or on the periphery of the smart pen.

The operation buttons 24 can be at least one of:
- Touch buttons
- Switches
- Rotation
- sliding

The skin conductance measurement smart pen system 12 may further includes a power source 26 to power electronic components of the smart pen. The power source 26 may include at least one of disposable batteries, plug-in rechargeable batteries, and a wireless inductive charging module.

The power source and the associated electronic circuit is capable of delivering up to 12 V and in a specific example of about 4 V.

In examples, the skin conductance measurement smart pen system 12 includes a temperature sensor 28.

In embodiments, one or more temperature sensors may be incorporated on the writing instrument 10.

In embodiments, one of the temperature sensors is located on the upper area of the pen body and is able to measure the environmental temperature.

In embodiments, another temperature sensor may be located at the finger positions adjacent or within to the electrodes in order to measure the finger temperature.

In embodiments, another temperature sensor may be located in the pen body near the electronic circuits (switching, measuring etc) in order to measure the circuit temperature.

Temperature values from one or all sensors may be used by the processor to calibrate accordingly the skin conductance readings.

Temperature sensors may be thermocouples, thermistors, resistance (RTD) or IR type.

In examples, the skin conductance measurement smart pen system 12 may include a humidity sensor 30. The humidity sensor may be located at the upper area of the pen body and is able to measure the environmental humidity. Humidity values may be used by the processor or algorithm to calibrate accordingly the skin conductance readings.

Humidity sensors may be one of the following types, a capacitive humidity sensor, a resistive humidity sensor, a thermal conductivity humidity sensor.

Besides the skin conductance measurement smart pen system 12 the writing instrument 10 includes the skin conductance changes software 14 which is capable of detecting changes in the skin conductance measured and of notifying the user accordingly.

One, some or all of the skin conductance software elements may be embedded in the smart writing instrument 10 or on an accompanying digital (operating) device.

The skin conductance measurement smart pen system 12 include the electronic circuits 31 which are capable of switching, measuring, calculating etc.

The skin conductance changes software 14 may include the following elements:
A skin conductance measurement algorithm 32 which is an algorithm that takes as input the measurements of the electric current generated by the sweat glands of the fingers during the application of the voltage pulses, process its evolution and calculates an average skin conductance over a specific timeframe.

A temperature and humidity calibration algorithm 34 which is an algorithm taking as input the measurement of one or all temperature sensors 28 and/or the humidity sensor 30. The temperature and humidity calibration algorithm 34 calibrates the current measured by the measuring electrodes 16.

An abnormal SC decrease algorithm 36 which is an algorithm that takes as input the calculated and calibrated skin conductance measurements and then compares the average skin conductance of an individual to a database of previous measurements of the specific individual and/or to a database of skin conductance similar cohorts of people with normal nerve function.

Depending on a set difference of skin conductance, the algorithm sends a signal to the visualization application characterizing the measurement as normal or abnormal i.e., when the skin conductance has significantly decreased.

A measurement may be considered as abnormal when a value of the measured skin conductance (SC) is lower than a 90% value of user's baseline average measurements or of reference data. For example, a skin conductance value of 71,2 µS may be classified as healthy while skin conductance value of 57,7 µS may be classified as indicative of Parkinson's Disease. A Siemens (S) is the SI unit of electrical conductance.

The skin conductance changes software 14 further includes an operation and data visualization application 38. This is a software that takes the measurements and the calculation results of the system algorithms and visualises the results to the user. It also permits the user to set specific parameters of the measurement and communicate them to the smart pen.

The above-mentioned algorithms may be executed on the writing instrument 10 or on an accompanying digital (operating) device. The operating device of the writing instrument 10 or smart pen may be a tablet, a smartphone, a smartwatch, a PC or any other suitable device capable of running the operation and visualisation application and/or the algorithms. The operating device is capable of communication wirelessly with the smart pen sending and receiving data.

In embodiments, the operating device may include, one or all the required algorithms and software for the processing and calculation of the data of the smart pen.

In embodiments, the operating device may include the user interface with which the user may interact with the smart pen and visualise the measurement results.

In embodiments, the operating device may alert the user with an audio, visual or haptic signal, in case of abnormal measurement data.

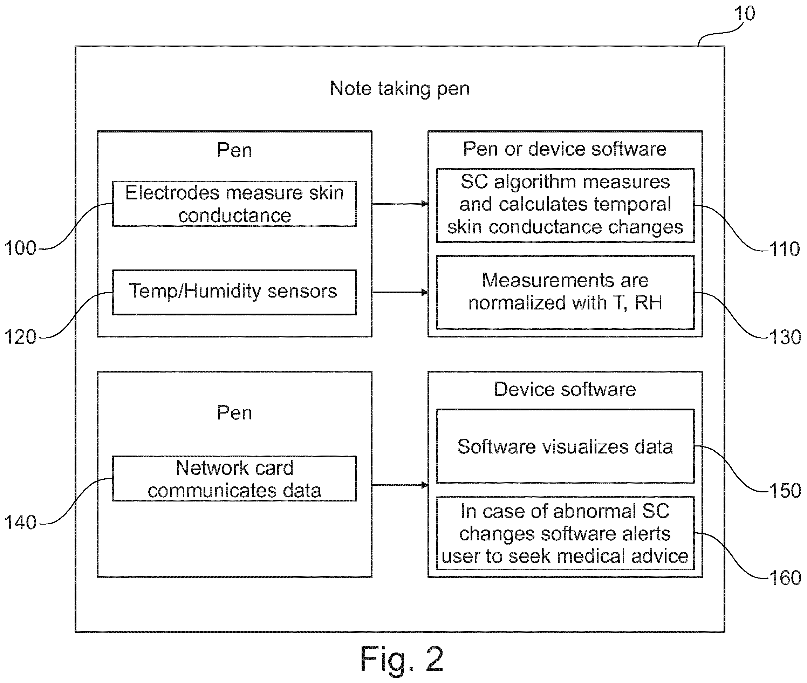

**Fig. 2** illustrates a process flow diagram of a writing instrument 10 according to the present disclosure. The writing instrument 10 can correspond to the writing instrument 10 as depicted in **Fig. 1****.**

In a first step or block 100 the electrodes 16 measure the skin conductance of the user of the writing instrument 10. This measurement data are measurement values that are passed to a block 110 in which the skin conductance measurement algorithm 32 calculates temporal changes of the skin conductance.

In a block 120, the temperature sensors 28 and the humidity sensor 30 measure e.g., skin temperature, ambient temperature and/or device temperature as well as ambient humidity. This data are measurement values that are passed to a block 130 in which the temperature and humidity calibration algorithm 34 normalizes or calibrates the values with temperature and/or humidity data. The blocks 110 and 130 can be implemented in the writing instrument 10 or in the operating device.

After the abnormal skin conductance decrease algorithm 36 has decided whether the measured data is normal or abnormal the network capability 22 like e.g. a network card communicates data in a block 140 to the device software. The device software can be implemented in the writing instrument 10 or in the operating device.

In a block 150, the operation and data visualization application 38 visualizes the data to the user and/or further persons. In cases of abnormal skin conductance changes the software alerts the user to seek medical advice.

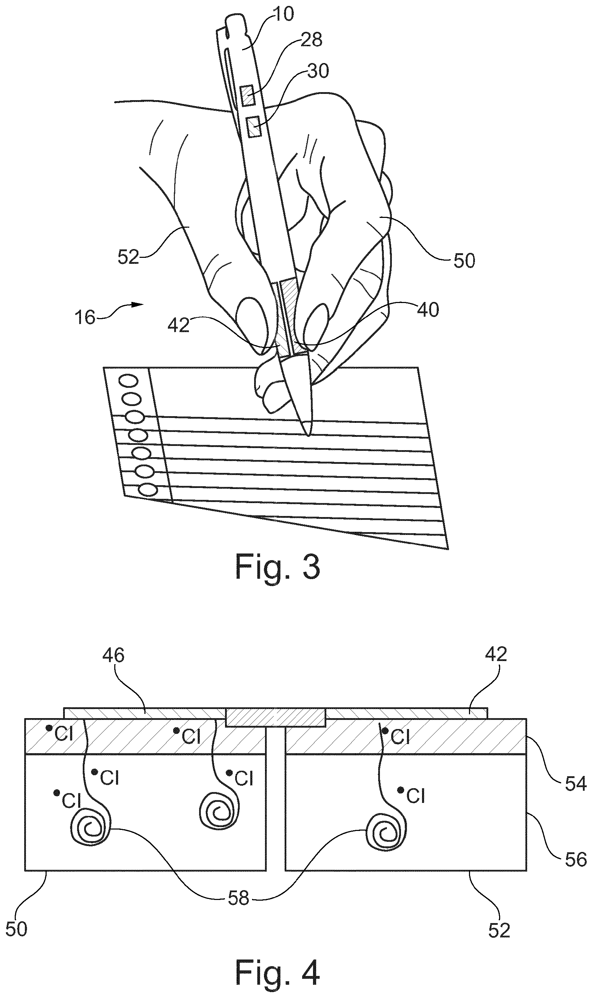

**Fig. 3** illustrates a perspective view of a writing instrument 10 according to the present disclosure. The writing instrument 10 can correspond to the writing instrument 10 as depicted **in** **Fig. 1** and **Fig. 2****.**

The writing instrument 10 includes electrodes 16 which are located in a gripping area close to the tip of the writing instrument 10. At an upper end of the writing instrument 10 opposite to the tip the ambient temperature sensor 28 and the humidity sensor 30 are located.

In Fig. 3, a user's hand is depicted holding the writing instrument in a typical 3 finger way. In this case, the index finger 50 is located on the positive electrode 40 and the thumb 52 is located on the negative electrode 42. Details of the measurement are explained in conjunction with Fig. 4 below.

**Fig. 4** illustrates a sectional view of an electrode arrangement of a writing instrument according to the present disclosure.

The positive electrode 40 and the negative electrode 42 are divided by a gap 44 in which an insulator is arranged so that the two electrodes 40, 42 are electrically insulated. The positive electrode 40 is in contact with the index finger 50 and the negative electrode 42 is in contact with the thumb 52. The electrodes 40, 42 are in contact with the epidermis 54 of the fingers 50, 52.

Below the epidermis 54 the dermis 56 is located. Sweat glands 58 are present in the dermis 56. Chloride ions Cl⁻ are present in the skin i.e., the epidermis 54 and the dermis 56.

Because the skin's stratum corneum acts like an electrical capacitor, when a low DC voltage stimulation e.g., ≤4V is applied via the electrodes 40, 42 the movement of chloride ions to the electrodes can only occur via the sweat ducts.

Thereby, a current is generated, relative to chloride ion flow supplied by the sweat glands and ducts. Active electrodes located on the finger rest position measure skin conductance on the basis of the current generated by the sweat glands and the voltage applied. The active electrodes may be the same electrodes 40, 42 used for applying the voltage stimulation.

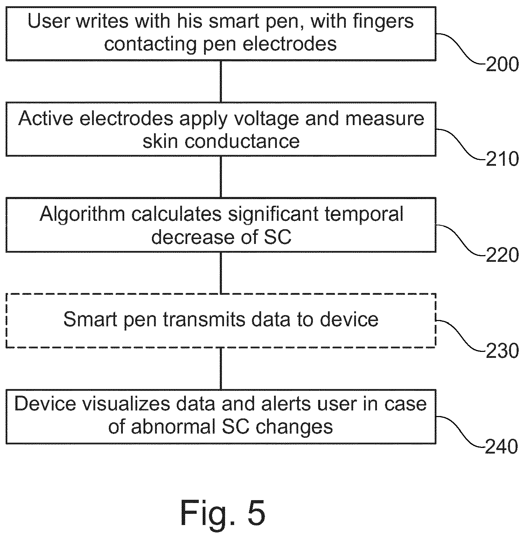

**Fig. 5** illustrates a method flow chart for measuring skin conductance of a writing instrument's user according to the present disclosure.

In a first step 200 of the method, the user has a writing session with the writing instrument 10. In the writing session, the user holds the smart pen to write. In normal operation the three fingers (thumb, index, middle) of his hand operating the pen firmly grip the smart pen in a specific location towards the pen tip.

In a second step 210, the active electrodes apply voltage and measure skin conductance. Thereby, the electrodes located at the smart pen gripping area induce a voltage at the skin of the three fingers. The applied voltage polarizes the sweat glands underneath the skin of the fingers which provide an ion flow driven to the electrodes by the applied voltage.

In other words, the second step 210 includes applying a DC voltage to user's fingers contacting the writing instrument during a writing session with the writing instrument; and measuring a current generated by reverse iontophoresis following applying the DC voltage.

In a third step 220, the algorithm calculates significant temporal decrease of the skin conductance (SC). This may include that the smart pen electrical circuit reads the resulting current. In examples, the temperature and humidity sensors measure temperature and humidity so that the system algorithms calculate and normalize the skin conductance values.

A measurement may be considered as abnormal when a value of the measured skin conductance (SC) is lower than a 90% value of average measurements or of reference data. For example, a skin conductance value of 71,2 µS may be classified as healthy while skin conductance value of 57,7 µS may be classified as indicative of Parkinson's Disease. A Siemens (S) is the SI unit of electrical conductance.

In other words, the third step 220 includes calculating an average skin conductance over a specific timeframe from the measured current; and comparing the average skin conductance to historical data from the same user or to reference data from a group of users. In examples, the third step 220 may include storing calculated average skin conductance data as historical data.

In an exemplary step 230, the smart pen transmits data to the operating device. It is also possible to keep the data at the writing instrument and to perform the next step at the writing instrument.

In an exemplary step 240, the device and/or the writing instrument visualizes data and alerts the user in case of abnormal SC changes. Alerting the user may be carried out even without the visualization of the data.

In other words, the exemplary step 240 includes providing an indication to the user in case that the skin conductance has decreased and/or the calculation results.

In examples, a biosensor located on the pen absorbs finger sweat and analyses health related sweat biomarkers. These biomarkers can be utilized in the calculation of step 220 and/or can be visualized as distinct data to the user in step 240.

The steps 100 to 160 as depicted in Fig. 2 can be included in the method of Fig. 5.

## Claims

1. A computer-implemented method for measuring skin conductance of a writing instrument's user, comprising:
- applying a DC voltage to user's fingers (50, 52) contacting the writing instrument (10) during a writing session with the writing instrument (10);
- measuring a current generated by reverse iontophoresis following applying the DC voltage;
- calculating an average skin conductance over a specific timeframe from the measured current;
- storing calculated average skin conductance data as historical data;
- comparing the average skin conductance to historical data from the same user or to reference data from a group of users; and
- providing an indication to the user in case that the skin conductance has decreased and/or the calculation results; and
- analyzing health related sweat biomarkers provided by a biosensor (18) of the writing instrument (10).

2. The method of claim 1, comprising:
- measuring at least one of ambient temperature, finger temperature, temperature of an electronic circuit of the writing instrument (10), and ambient humidity;
- calibrating the average skin conductance with at least one of the measured temperatures and the measured ambient humidity.

3. The method of one of the preceding claims, wherein the DC voltage is applied in voltage pulses each comprising duration ranges from 0,1 seconds to 10 seconds.

4. The method of one of the preceding claims, wherein a skin conductance is calculated based on a number of sequential voltage pulses in a range from 5 to 50.

5. The method of one of the preceding claims, wherein the indication and/or the calculation results are provided via a user interface of the writing instrument (10).

6. The method of one of the preceding claims, wherein the indication and/or the calculation results are provided via a wireless communication system (22) to an external device having an interface.

7. A writing instrument (10) for measuring skin conductance of its user, comprising:
- at least two electrodes (40, 42) configured to apply a DC voltage to user's fingers (50, 52) contacting the writing instrument (10) during a writing session with the writing instrument (10);
- one or more sensors (28) configured to measuring a current generated by reverse iontophoresis following applying the DC voltage;
- a computer system (20) configured to execute the computer-implemented method for measuring skin conductance according to one of the preceding claims;
- a storage system configured to store calculated average skin conductance data as historical pressure data; and
- a communication system configured to provide an indication to the user in case that the skin conductance has decreased and/or the calculation results;
- a biosensor (18) configured to analyze health related sweat biomarkers.

8. The writing instrument (10) of claim 7, comprising:
- at least one temperature sensor (28) configured to measure at least one of ambient temperature, finger (50) temperature, temperature of an electronic circuit of the writing instrument (10);
- a humidity sensor (30) configured to measure ambient humidity.

9. The writing instrument (10) of one of claims 7 to 8, wherein the electrodes (40, 42) are located at a grip area of the writing instrument (10).

10. The writing instrument (10) of one of claims 7 to 9, wherein two of the electrodes (40, 42) are the anode and the cathode electrodes during a measurement and wherein a third or more electrodes (16) are passive electrodes and are connected with high impedance configured to measure the potential reached by the user's body.

11. The writing instrument (10) of one of claims 7 to 10, wherein a gap area (44) made of a non-conductive material is provided between two electrodes (16).

12. The writing instrument (10) of claim 11, wherein a surface of the gap (44) area is elevated or receded relative to a surface of the electrodes (16).

13. The writing instrument (10) of one of claims 7 to 12, comprising:
- a user interface configured to receive input from a user and/or to provide information to the user such as the indication and/or the calculation results; and/or
- a wireless communication system (22) configured to provide the indication and/or the calculation results via an external device having an interface.

## Patentansprüche

1. Computerimplementiertes Verfahren zum Messen einer Hautleitfähigkeit eines Benutzers eines Schreibinstruments, umfassend:
- Anlegen einer Gleichspannung an die Finger (50, 52) des Benutzers, die das Schreibinstrument (10) während einer Schreibsitzung mit dem Schreibinstrument (10) berühren;
- Messen eines Stroms, der durch Umkehriontophorese erzeugt wird, nach dem Anlegen der Gleichspannung;
- Berechnen einer durchschnittlichen Hautleitfähigkeit über einen spezifischen Zeitraum aus dem gemessenen Strom;
- Speichern von berechneten durchschnittlichen Hautleitfähigkeitsdaten als historische Daten;
- Vergleichen der durchschnittlichen Hautleitfähigkeit mit historischen Daten von dem gleichen Benutzer oder mit Referenzdaten von einer Gruppe von Benutzern; und
- Bereitstellen einer Angabe für den Benutzer im Falle, dass die Hautleitfähigkeit abgenommen hat, und/oder der Berechnungsergebnisse; und
- Analysieren gesundheitsbezogener Schweißbiomarker, die durch einen Biosensor (18) des Schreibinstruments (10) bereitgestellt werden.

2. Verfahren nach Anspruch 1, umfassend:
- Messen mindestens einer von einer Umgebungstemperatur, einer Temperatur der Finger, einer Temperatur einer elektronischen Schaltung des Schreibinstruments (10) und einer Umgebungsfeuchtigkeit;
- Kalibrieren der durchschnittlichen Hautleitfähigkeit mit mindestens einer der gemessenen Temperaturen und der gemessenen Umgebungsfeuchtigkeit.

3. Verfahren nach einem der vorstehenden Ansprüche, wobei die Gleichspannung in Spannungsimpulsen, jeweils umfassend Dauerbereiche von 0,1 Sekunden bis 10 Sekunden, angelegt wird.

4. Verfahren nach einem der vorstehenden Ansprüche, wobei eine Hautleitfähigkeit basierend auf einer Anzahl von aufeinanderfolgenden Spannungsimpulsen in einem Bereich von 5 bis 50 berechnet wird.

5. Verfahren nach einem der vorstehenden Ansprüche, wobei die Angabe und/oder die Berechnungsergebnisse mittels einer Benutzeroberfläche des Schreibinstruments (10) bereitgestellt werden.

6. Verfahren nach einem der vorstehenden Ansprüche, wobei die Angabe und/oder die Berechnungsergebnisse mittels eines drahtlosen Kommunikationssystems (22) einer externen Vorrichtung, die eine Schnittstelle aufweist, bereitgestellt werden.

7. Schreibinstrument (10) zum Messen der Hautleitfähigkeit seines Benutzers, umfassend:
- mindestens zwei Elektroden (40, 42), die konfiguriert sind, um eine Gleichspannung an die Finger (50, 52) des Benutzers, die während einer Schreibsitzung mit dem Schreibinstrument (10) das Schreibinstrument (10) berühren, anzulegen;
- einen oder mehrere Sensoren (28), die konfiguriert sind, um einen Strom, der durch Umkehriontophorese erzeugt wird, nach dem Anlegen der Gleichspannung zu messen;
- ein Computersystem (20), das konfiguriert ist, um das computerimplementierte Verfahren zum Messen der Hautleitfähigkeit nach einem der vorstehenden Ansprüche auszuführen;
- ein Speichersystem, das konfiguriert ist, um die berechnete durchschnittliche Hautleitfähigkeitsdaten als historische Druckdaten zu speichern; und
- ein Kommunikationssystem, das konfiguriert ist, um dem Benutzer eine Angabe im Falle, dass die Hautleitfähigkeit abgenommen hat, und/oder die Berechnungsergebnisse bereitzustellen;
- einen Biosensor (18), der konfiguriert ist, um gesundheitsbezogene Schweißbiomarker zu analysieren.

8. Schreibinstrument (10) nach Anspruch 7, umfassend:
- mindestens einen Temperatursensor (28), der konfiguriert ist, um mindestens eine von Umgebungstemperatur, der Temperatur der Finger (50), der Temperatur einer elektronischen Schaltung des Schreibinstruments (10) zu messen;
- einen Feuchtigkeitssensor (30), der konfiguriert ist, um die Umgebungsfeuchtigkeit zu messen.

9. Schreibinstrument (10) nach einem der Ansprüche 7 bis 8, wobei sich die Elektroden (40, 42) an einer Grifffläche des Schreibinstruments (10) befinden.

10. Schreibinstrument (10) nach einem der Ansprüche 7 bis 9, wobei zwei der Elektroden (40, 42) während einer Messung die Anoden- und die Kathodenelektroden sind und wobei eine dritte oder mehr Elektroden (16) passive Elektroden sind und mit hoher Impedanz verbunden sind, die konfiguriert sind, um das Potenzial, das durch den Körper des Benutzers erreicht wird, zu messen.

11. Schreibinstrument (10) nach einem der Ansprüche 7 bis 10, wobei zwischen zwei Elektroden (16) eine Spaltfläche (44), die aus einem nichtleitenden Material hergestellt ist, bereitgestellt ist.

12. Schreibinstrument (10) nach Anspruch 11, wobei eine Oberfläche der Abstandsfläche (44) relativ zu einer Oberfläche der Elektroden (16) erhöht oder zurückgesetzt ist.

13. Schreibinstrument (10) nach einem der Ansprüche 7 bis 12, umfassend:
- eine Benutzerschnittstelle, die konfiguriert ist, um eine Eingabe von einem Benutzer zu empfangen und/oder um dem Benutzer Informationen, wie die Angabe und/oder die Berechnungsergebnisse, bereitzustellen; und/oder
- ein drahtloses Kommunikationssystem (22), das konfiguriert ist, um die Angabe und/oder die Berechnungsergebnisse mittels einer externen Vorrichtung, die eine Schnittstelle aufweist, bereitzustellen.

## Revendications

1. Procédé mis en œuvre par ordinateur destiné à mesurer une conductance cutanée d'un utilisateur d'un instrument d'écriture, comprenant :
- l'application d'une tension CC à des doigts (50, 52) de l'utilisateur en contact avec l'instrument d'écriture (10) pendant une séance d'écriture avec l'instrument d'écriture (10) ;
- la mesure d'un courant généré par iontophorèse inverse suite à l'application de la tension CC ;
- le calcul d'une conductance cutanée moyenne sur une période spécifique à partir du courant mesuré ;
- le stockage de données de conductance cutanée moyenne calculée en tant que données historiques ;
- la comparaison de la conductance cutanée moyenne à des données historiques du même utilisateur ou à des données de référence d'un groupe d'utilisateurs ; et
- la fourniture d'une indication à l'utilisateur dans le cas où la conductance cutanée a diminué et/ou des résultats de calcul ; et
- l'analyse de biomarqueurs de sueur liés à la santé fournis par un biocapteur (18) de l'instrument d'écriture (10).

2. Procédé selon la revendication 1, comprenant :
- la mesure d'au moins l'une parmi une température ambiante, une température de doigt, une température d'un circuit électronique de l'instrument d'écriture (10), et une humidité ambiante;
- l'étalonnage de la conductance cutanée moyenne avec au moins l'une des températures mesurées et de l'humidité ambiante mesurée.

3. Procédé selon l'une des revendications précédentes, dans lequel la tension CC est appliquée sous forme d'impulsions de tension présentant chacune une durée comprise dans une plage allant de 0,1 seconde à 10 secondes.

4. Procédé selon l'une des revendications précédentes, dans lequel la conductance cutanée est calculée sur la base d'un nombre d'impulsions de tension séquentielles compris dans une plage allant de 5 à 50.

5. Procédé selon l'une des revendications précédentes, dans lequel l'indication et/ou les résultats de calcul sont fournis par l'intermédiaire d'une interface utilisateur de l'instrument d'écriture (10).

6. Procédé selon l'une des revendications précédentes, dans lequel l'indication et/ou les résultats de calcul sont fournis par l'intermédiaire d'un système de communication sans fil (22) à un dispositif externe ayant une interface.

7. Instrument d'écriture (10) destiné à mesurer une conductance cutanée de son utilisateur, comprenant :
- au moins deux électrodes (40, 42) configurées pour appliquer une tension CC à des doigts (50, 52) de l'utilisateur en contact avec l'instrument d'écriture (10) pendant une séance d'écriture avec l'instrument d'écriture (10) ;
- un ou plusieurs capteurs (28) configurés pour mesurer un courant généré par iontophorèse inverse suite à l'application de la tension CC ;
- un système informatique (20) configuré pour exécuter le procédé mis en œuvre par ordinateur destiné à mesurer une conductance cutanée selon l'une des revendications précédentes ;
- un système de stockage configuré pour stocker des données de conductance cutanée moyenne calculée en tant que données de pression historiques ; et
- un système de communication configuré pour fournir une indication à l'utilisateur dans le cas où la conductance cutanée a diminué et/ou les résultats de calcul ;
- un biocapteur (18) configuré pour analyser des biomarqueurs de sueur liés à la santé.

8. Instrument d'écriture (10) selon la revendication 7, comprenant :
- au moins un capteur de température (28) configuré pour mesurer au moins l'une parmi une température ambiante, une température de doigt (50), une température d'un circuit électronique de l'instrument d'écriture (10) ;
- un capteur d'humidité (30) configuré pour mesurer une humidité ambiante.

9. Instrument d'écriture (10) selon l'une des revendications 7 à 8, dans lequel les électrodes (40, 42) sont situées au niveau d'une zone de préhension de l'instrument d'écriture (10).

10. Instrument d'écriture (10) selon l'une des revendications 7 à 9, dans lequel deux des électrodes (40, 42) sont les électrodes d'anode et de cathode pendant une mesure et dans lequel une troisième ou plus électrodes (16) sont des électrodes passives et sont connectées avec une haute impédance configurée pour mesurer le potentiel atteint par le corps de l'utilisateur.

11. Instrument d'écriture (10) selon l'une des revendications 7 à 10, dans lequel une zone d'espace (44) faite d'un matériau non conducteur est prévue entre deux électrodes (16).

12. Instrument d'écriture (10) selon la revendication 11, dans lequel une surface de la zone d'espace (44) est surélevée ou reculée par rapport à une surface des électrodes (16).

13. Instrument d'écriture (10) selon l'une des revendications 7 à 12, comprenant :
- une interface utilisateur configurée pour recevoir une entrée d'un utilisateur et/ou pour fournir des informations à l'utilisateur telles que l'indication et/ou les résultats de calcul ; et/ou
- un système de communication sans fil (22) configuré pour fournir l'indication et/ou les résultats de calcul par l'intermédiaire d'un dispositif externe ayant une interface.
